# EUROPÄISCHE PATENTSCHRIFT

(11) **EP 2 701 671 B1**
(45) Veröffentlichungstag und Bekanntmachung des Hinweises auf die Patenterteilung: **16.03.2016**
(21) Anmeldenummer: 12716421.8
(22) Anmeldetag: 24.04.2012
(51) Int. Cl.: A61K 8/34, A61K 8/365, A61K 8/37, A61K 8/44, A61Q 9/02, A61Q 17/04, A61Q 19/00, A61Q 19/04, A61Q 19/06, A61K 8/04, A61K 8/06, A61K 8/92

(54) **OW-EMULSIONSKONZENTRAT, VERFAHREN ZU DESSEN HERSTELLUNG UND DIESES ENTHALTENDES HAUTKOSMETIKUM**
OW EMULSION CONCENTRATE, PROCESS FOR THE MANUFACTURE THEREOF AND COSMETIC FOR THE SKIN CONTAINING SAID CONCENTRATE
CONCENTRE D'UNE OW EMULSION, PROCES POUR SA FABRICATION ET& xA;PRODUIT DE BEAUTE POUR LA PEAU CONTENANT CE CONCENTRE

(30) Priorität: 28.04.2011 DE 102011018924
(43) Veröffentlichungstag der Anmeldung: 05.03.2014
(73) Patentinhaber: TUNAP Industrie Chemie GmbH & Co. Produktions KG, 82515 Wolfratshausen (DE)
(72) Erfinder: SPRÜGEL, Friedrich, 81825 München (DE); BROTT, Roland, 09221 Neukirchen (DE)
(74) Vertreter: Ter Meer Steinmeister & Partner
(86) Internationale Anmeldenummer: PCT/EP2012/057444
(87) Internationale Veröffentlichungsnummer: WO 2012/146576

(56) Entgegenhaltungen:
- EP-A1- 2 243 462
- WO-A2-99/08649
- DE-A1- 10 344 888
- "Symbiomuls - Product information", , 12. Oktober 2009 (2009-10-12), XP55034043, Gefunden im Internet: URL:http://www.dr-straetmans.de/de/produkt e/produktbeschreibung_symbiomuls_gc.php [gefunden am 2012-07-27]

## Beschreibung

### Gebiet der Erfindung

Die vorliegende Erfindung betrifft ein pH-Wert-stabiles OW-Emulsionskonzentrat zur Herstellung von Hautkosmetika, ein Verfahren zur Herstellung des OW-Emulsionskonzentrats sowie Hautkosmetika in Form von Flüssig- oder Schaumprodukten, welche auf Basis des OW-Emulsionskonzentrats hergestellt werden können.

### Stand der Technik

Der Hauttyp eines Menschen ist vor allem genetisch bedingt und wird durch die natürliche Talgproduktion bestimmt. Je nach dem wie ausgeprägt diese ist, zeigt sich die Haut als normal, trocken oder auch sehr trocken. Vielfältige innere und äußere Einflüsse können sich auf das Hauterscheinungsbild niederschlagen und den Hautzustand des Körpers verändern. Zur Pflege und zum Schutz der Haut werden vielfältige Hautkosmetika verwendet.

Die Verwendung von 2-Phasen-Emulsionen aus hydrophoben Komponenten einerseits und hydrophilen Komponenten andererseits zur Herstellung von Schaumpräparaten für die Hautpflege ist bereits bekannt. Beispielsweise beschreibt die EP 1 014 916 B1 eine Schaum-Hautcreme, die erhalten wird durch Herstellung einer hydrophoben Phase durch Aufschmelzen bei 75°C einer Fettsäuren und Emulgatoren enthaltenden Mischung, Zugabe zu einer auf 75°C temperierten wässrigen Mischung, enthaltend Moisturizer und/oder Emulgatoren und/oder Hautpflegeadditive, Rühren der Mischung bei 75°C, danach unter ständigem Rühren Abkühlung auf 30-40°C, Einstellung des pH-Wertes auf 7,6 bis 8,2 mit einer hautverträglichen basischen organischen Verbindung und Abfüllung der erhaltenen Mischung in Darreichungsformen unter Zugabe eines Treibgases. Diese Schaum-Hautcreme kann als Hautschutzcreme oder zur Linderung von dermatologischen Erkrankungen oder, wenn der wässrigen Phase zusätzliche hydratisierende Mittel zugesetzt werden, zur Prophylaxe und Therapie des diabetischen Fußes eingesetzt werden.

Die in EP 1 014 916 B1 beschriebene Schaum-Hautcreme ist mit verschiedenen Nachteilen verbunden. Zu einen erfordert die gesamte Herstellung der Schaum-Hautcreme einen hohen Energieaufwand, da es notwendig ist, sowohl die hydrophobe als auch die hydrophile Phase auf 75°C zu erwärmen und über längere Zeit bei dieser Temperatur unter Rühren zu halten. Weiterhin muss der pH-Wert der Schaum-Hautcreme mittels einer basischen organischen Verbindung auf einen Wert zwischen 7,6 und 8,2 eingestellt werden. Hierzu wird 2-Amino-2-methyl-1-propanol verwendet, bei dem es sich ebenfalls um ein Konservierungsmittel handelt. Die Einstellung eines basischen pH-Wertes von 7,6 bis 8,2 wirkt jedoch dem natürlichen Säureschutzmantel der Haut, der einem pH-Wert von etwa 4 bis 6,5 entspricht und durch körpereigene saure Substanzen in Schweiß, Talg und Hornzellen zustande kommt, entgegen. Die Aufgabe dieses Säureschutzmantels besteht darin, schädliche Mikroorganismen sowie negative Umwelteinflüsse abzuwehren und dadurch die Haut vor Infektionen, Reizung, Allergie und Austrocknung zu schützen. Basische Kosmetikpräparate können jedoch - zumindest vorübergehend - zu einer teilweisen Zerstörung des Säureschutzmantels führen, so dass schädliche Mikroorganismen sowie negative Umwelteinflüsse nicht mehr in ausreichendem Maße abgewehrt werden können. Des weiteren enthalten die Schaum-Hautcremes gemäß EP 1 014 916 B1 freie Fettsäuren, die zur Instabilität neigen, so dass im Endeffekt die hergestellten Kosmetikpräparate nicht pH-Wert-stabil sind. Schließlich enthalten die gemäß EP 1 014 916 B1 hergestellten Schaum-Hautcremepräparate vielfach Rohstoffe auf Mineralölbasis und somit auf Basis nichtnachwachsender Rohstoffe.

### Aufgabe der Erfindung

Der Erfindung liegt daher die Aufgabe zugrunde, unter Überwindung der Nachteile des Standes der Technik pH-Wert-stabile OW-Emulsionskonzentrate zur Verfügung zu stellen, welche den Säureschutzmantel der Haut nicht angreifen und die im wesentlichen aus natürlichen bzw. nachwachsenden Rohstoffen hergestellt werden und sich zu verschiedenen Hautkosmetika in Form von Flüssig- oder Schaumpräparaten weiterverarbeiten lassen.

Weiterhin liegt der Erfindung die Aufgabe zugrunde, ein kostengünstiges und einfaches Verfahren zur Herstellung solcher OW-Emulsionskonzentrate zur Verfügung zu stellen.

### Zusammenfassung der Erfindung

Die vorstehende Aufgabe wird erfindungsgemäß gelöst durch ein pH-Wert-stabiles OW-Emulsionskonzentrat gemäß Anspruch 1, ein Verfahren zu dessen Herstellung gemäß Anspruch 6 sowie ein Hautkosmetikum gemäß Anspruch 7. Bevorzugte bzw. besonders zweckmäßige Ausführungsformen des Anmeldungsgegenstandes sind in den Unteransprüchen angegeben.

Gegenstand der Erfindung ist somit ein pH-Wert-stabiles, keine freien Fettsäuren aufweisendes OW-Emulsionskonzentrat zur Herstellung von Hautkosmetika, umfassend
A) in der Ölphase
   a) 4-12 Gew.-%, bezogen auf das Emulsionskonzentrat, einer Emulgatormischung auf Basis von Glycerylstearatcitrat, Cetearylalkohol und Glycerylcaprylat,
   b) bis zu 2 Gew.-%, vorzugsweise 0,5-2 Gew.-%, bezogen auf das Emulsionskonzentrat, eines Caprylsäureesters,
   c) wahlweise native und/oder synthetische kosmetische Öle,
   d) wahlweise Fettalkohole mit 12-20 C-Atomen,
   e) wahlweise Pflanzenbutter,
B) in der Wasserphase
   a) 2-6 Gew.-%, bezogen auf das Emulsionskonzentrat, eines oder mehrerer schaumgebenden Tenside,
   b) wahlweise Moisturizer,
   c) Milchsäure und/oder deren Salze zur Einstellung des pH-Wertes des Emulsionskonzentrats auf 4,5 bis 6.

Gegenstand der Erfindung ist ebenso ein Verfahren zur Herstellung eines solchen OW-Emulsionskonzentrats, umfassend die Schritte:
i) Vorlegen der auf eine Temperatur von 30-50°C erwärmten und homogenisierten Ölphase
ii) Einrühren der eine Temperatur von 20-30°C aufweisenden Wasserphase, um durch Phasenumkehr ein OW-Emulsionskonzentrat zu bilden,
iii) Abkühlenlassen des OW-Emulsionskonzentrats unter Rühren auf Raumtemperatur.

Gegenstand der Erfindung ist weiterhin ein Hautkosmetikum, umfassend ein erfindungsgemäßes OW-Emulsionskonzentrat, ein Hautpflegeadditiv und/oder Hautschutzadditiv, zusätzlich Wasser zur Einstellung der gewünschten Konzentration der Bestandteile sowie gegebenenfalls ein Treibgas.

Überraschenderweise hat sich gemäß der Erfindung gezeigt, dass mit einem OW-Emulsionskonzentrat gemäß Anspruch 1 eine pH-Wert-stabile Grundmischung vorgesehen werden kann, deren Komponenten auf Basis natürlicher bzw. nachwachsender Rohstoffe erhalten werden, die keine zusätzlichen Konservierungsstoffe erfordert und welche den natürlichen Säureschutzmantel der Haut nicht zerstört. Diese Grundmischung eignet sich für die Weiterverarbeitung zu verschiedenen Hautkosmetika in Flüssig- oder Schaumform, wobei letztere auch als "Schaumaerosole" bezeichnet werden. Insbesondere eignet sich ein erfindungsgemäßes OW-Emulsionskonzentrat zur Weiterverarbeitung zu einem Hautkosmetikum für die Fußpflege, insbesondere für die prophylaktische und therapeutische Behandlung des diabetischen Fußes, wobei in diesem Fall als Hautpflegeadditiv ein hydratisierendes Mittel, insbesondere Harnstoff, sowie strukturgebende Stoffe auf Basis von Cellulosederivaten, beispielsweise auf Basis von Stärkepulvern aus Mais, Reis, Kartoffel oder Maniok, zugesetzt werden.

### Detaillierte Beschreibung der Erfindung

Die vorliegende Erfindung wird anhand bevorzugter Ausführungsformen nachfolgend näher erläutert.

Wie oben erwähnt, umfasst die Ölphase des erfindungsgemäßen OW-Emulsionskonzentrats
a) 4-12 Gew.-%, bezogen auf das Emulsionskonzentrat, einer Emulgatormischung auf Basis von Glycerylstearatcitrat, Cetearylalkohol und Glycerylcaprylat,
b) bis zu 2 Gew.-%, vorzugsweise 0,5-2 Gew.-%, bezogen auf das Emulsionskonzentrat, eines Caprylsäureesters,
c) wahlweise native und/oder synthetische kosmetische Öle,
d) wahlweise Fettalkohole mit 12-20 C-Atomen,
e) wahlweise Pflanzenbutter.

Für die Emulgatormischung a) kann ein im Handel unter der Bezeichnung symbio®muls GC erhältliches Präparat eingesetzt werden. Hierbei handelt es sich um eine aus natürlichen Materialien hergestellte Emulgatormischung, wobei die Komponenten Glycerylstearatcitrat, Cetearylalkohol und Glycerylcaprylat geeigneterweise im Gewichtsverhältnis von etwa 16:16:1 vorliegen.

Als Caprylsäureester b) wird vorzugsweise Glycerylcaprylat eingesetzt, beispielsweise das im Handel erhältliche Produkt Dermosoft® GMCY. Ein weiteres Beispiel ist Propylheptylcaprylat. Solche Caprylsäureester wirken biologisch stabilisierend und haben neben ihrer guten Hautverträglichkeit Wirkung gegen Hefen und Bakterien.

Das wahlweise enthaltene native kosmetische Öl wird vorzugsweise aus der Gruppe gewählt, bestehend aus Jojobaöl, Sanddornfruchtfleischöl, Avocadoöl und Mandelöl. Das wahlweise enthaltene synthetische kosmetische Öl ist vorzugsweise ein Fettsäureester, weiter vorzugsweise gewählt aus der Gruppe, bestehend aus Cetylpalmitat, Isopropylmyristat, Isopropylpalmitat und Decyloleat.

Die wahlweise enthaltenen Fettalkohole d) sind solche mit 12-20 C-Atomen, beispielsweise Cetylalkohol (C16) und Stearylalkohol (C18).

Als wahlweise enthaltene Pflanzenbutter e) wird vorzugsweise Sheabutter verwendet.

Sämtliche der oben genannten Komponenten basieren auf natürlichen bzw. nachwachsenden Rohstoffen.

Falls erwünscht, können der Ölphase auch synthetische Komponenten, wie Siliconöle und/oder Paraffinöle zugesetzt werden. Beispiele für Siliconöle sind Dimethicone und Cyclomethicone.

Die Wasserphase des erfindungsgemäßen OW-Emulsionskonzentrats umfasst folgende Komponenten:
a) 2-6 Gew.-%, bezogen auf das Emulsionskonzentrat, eines oder mehrerer schaumgebenden Tenside,
b) wahlweise Moisturizer,
c) Milchsäure und/oder deren Salze zur Einstellung des pH-Wertes des Emulsionskonzentrats auf 4,5 bis 6.

Die schaumgebenden Tenside sind vorzugsweise aus der Gruppe gewählt, bestehend aus Alkylsarcosinaten, Alkoylsarcosinaten, Alkylglutamaten, Alkoylglutamaten und deren Salzen.

Zusätzlich können die erfindungsgemäßen OW-Emulsionskonzentrate nach Bedarf hautverträgliche anionenaktive Tenside, wie Fettalkoholsulfate und Sulfosuccinate, Amphotenside, wie Betaine sowie neutrale Tenside, wie Zuckertenside enthalten. Vorzugsweise sind sämtliche dieser Tenside auf Basis natürlicher Rohstoffe hergestellt.

Die Moisturizer b) sind vorzugsweise gewählt aus der Gruppe, bestehend aus mehrwertigen Alkoholen und Zuckeralkoholen, vorzugsweise aus der Gruppe, bestehend aus Propylenglykol, Glycerin und Sorbitol.

Die Milchsäure und/oder deren Salze, beispielsweise Natriumlactat, dienen zur Einstellung des pH-Wertes des Emulsionskonzentrats auf Werte im Bereich von 4,5 bis 6, vorzugsweise etwa 5,5, was dem durchschnittlichen pH-Wert des natürlichen Säureschutzmantels der Haut entspricht.

Der Feststoffgehalt der Wasserphase beträgt üblicherweise 25-35 Gew.-%, bezogen auf die Wasserphase. Der Anteil schaumgebender Tenside beträgt üblicherweise 5-15 Gew.-% und der Anteil an Moisturizer beträgt beispielsweise 7-28 Gew.-%, jeweils bezogen auf die Wasserphase.

Innerhalb des erfindungsgemäßen OW-Emulsionskonzentrats beträgt der Anteil der Ölphase üblicherweise 30-50 Gew.-%, bezogen auf das Emulsionskonzentrat.

Die Herstellung des erfindungsgemäßen OW-Emulsionskonzentrats ist einfach und kostengünstig und erfordert nicht das Aufheizen und Halten der jeweiligen Phasen auf einer Temperatur von 75°C wie im Falle der EP 1 014 916 B1.

Das Einrühren der eine Temperatur von 20-30°C aufweisenden Wasserphase in die auf 30-50°C erwärmte und homogenisierte Ölphase erfolgt vorzugsweise unter Verwendung eines Hochgeschwindigkeitsrührers mit Umdrehungszahlen von geeigneterweise 2000-6000 U/min, um durch Phasenumkehr ein OW-Emulsionskonzentrat zu bilden.

Das Einrühren der vorgemischten Wasserphase in die vorgemischte Ölphase erfolgt vorzugsweise portionsweise unter Anlegen eines hohen Schergefälles mit Hilfe eines Rührers bei den oben genannten Umdrehungszahlen, so dass nach anfänglichem Verdicken der vorgelegten Ölphase mit steigender Menge der Wasserphase eine Phasenumkehr bewirkt wird bei gleichzeitiger Verringerung der Verdickung unter Bildung einer OW-Emulsion.

Falls erforderlich, erfolgt nach Herstellung des OW-Emulsionskonzentrats eine Kontrolleinstellung des pH-Wertes, um dieses mit Milchsäure und/oder deren Salze auf den erwünschten pH-Wert von 4,5 bis 6 einzustellen.

Das auf diese Weise erhaltene OW-Emulsionskonzentrat ist eine pH-Wert-stabile Basisemulsion mit sehr hoher Verträglichkeit für die Zugabe von weiteren spezifischen, multifunktionellen Hautpflegewirkstoffen, wobei Hautkosmetika mit gewünschten Endviskositäten erhalten werden.

Das erfindungsgemäße OW-Emulsionskonzentrat eignet sich daher als Grundmischung zur Weiterverarbeitung zu verschiedenen Hautkosmetika, die im einfachsten Falle ein erfindungsgemäßes OW-Emulsionskonzentrat, ein Hautpflegeadditiv und/oder Hautschutzadditiv, zusätzlich Wasser zur Einstellung der gewünschten Konzentration der Bestandteile und der gewünschten Endviskosität sowie gegebenenfalls mindestens ein Treibgas enthalten.

Das Hautpflegeadditiv wird geeigneterweise aus der Gruppe gewählt, bestehend aus rückfettenden Stoffen, Pro-Age-Mitteln, Selbstbräunungsmitteln, Anticellulitismitteln, Whiteningmitteln, Fußpflegemitteln, Hyaluronsäure, kühlenden oder wärmenden Mitteln, Stärken, phosphatveresterten Stärken auf Reis- und/oder Maisbasis, modifizierten Xanthangummis und hydratisierenden Mitteln.

Vorzugsweise wird das Hautschutzadditiv aus der Gruppe gewählt, bestehend aus antibakteriellen Mitteln, antimykotischen Mitteln, Sonnenschutzmitteln auf organischer und/oder anorganischer Basis und Repellentwirkstoffen.

In einer besonders bevorzugten Ausführungsform dient das erfindungsgemäße OW-Emulsionskonzentrat zur Herstellung eines Hautkosmetikums für die Fußpflege, insbesondere für die prophylaktische und therapeutische Behandlung des diabetischen Fußes, enthaltend hydratisierende Mittel, vorzugsweise Harnstoff, sowie strukturgebende Stoffe auf Basis von Cellulosederivaten.

Die Oberschicht der Haut enthält von Natur aus Harnstoff in und zwischen den Zellen. Sinkt der Anteil an Harnstoff, trocknet die Haut aus. Wasser löst zusätzlich bei jedem Waschen mehr Harnstoff aus der oberen Hautschicht. Harnstoff verfügt über mehrere vorteilhafte Eigenschaften. Er bindet viel Feuchtigkeit in der Hornschicht und lockert sie. Des weiteren regt Harnstoff die Hornhaut an, tote Zellen schneller abzustoßen, wodurch sich Schrunden zurückbilden.

Das aus dem erfindungsgemäßen Emulsionkonzentrat herstellbare Hautkosmetikum kann ein Flüssigpräparat sein, vorzugsweise jedoch ein Schaumaerosol, enthaltend als Treibgas Dimethylether und/oder Kohlenwasserstofftreibmittel, sowie gegebenenfalls Stickstoff und/oder CO₂ zur Druckstufeneinstellung. Besonders bevorzugt ist ein sprühbares, aufschäumendes Aerosol, abgefüllt in einer 2-Kammer [bag-on-valve (BOV)] Aerosoldruckdose oder einer 3-Kammer [bag-in-bag-on-valve (BBOV)] Aerosoldruckdose. Hierbei ist es bevorzugt, dass die innere Kammer der Spraydose (BOV oder BBOV) mit dem gewählten Wirkstoffgemisch gemäß den Beispielen und Dimethylether und/oder Kohlenwasserstofftreibmittel eine niedere Druckstufe hat, welche durch die äußere Kammer und CO₂ und/oder Stickstoff als höhere Druckstufe ausgesprüht und dann das Wirkstoffgemisch durch Ausgasen des Treibgases aufgeschäumt wird.

### Bevorzugte Ausführungsformen

Die Erfindung wird anhand der nachfolgenden Ausführungsbeispiele in Form von verschiedenen Rezepturen näher erläutert.

### Beispiel 1

**Rezeptur Ölphase**

| Bestandteile | chemische Bezeichnung | Gew.-% |
|---|---|---|
| Dermofeel sensolv | Isoamyllaurat | 13,95 |
| Lanette 16 | Cetylalkohol | 9,30 |
| Dermosoft GMCY | Glycerylcaprylat | 4,65 |
| Cetiol sensoft | Propylheptylcaprylat | 13,95 |
| Cetiol PGL | Hexyldecanol | 6,98 |
| | Hexyldecyllaurat | 6,98 |
| Shea Butter | Buryrospermum Parkii Butter | 4,65 |
| Mais PO4 PH "B" | Distärkephosphat | 9,30 |
| Symbiomuls GC | Glvcerylstearatcitrat | 14,66 |
| | Cetearylalkohol | 14,66 |
| | Glycerylcaprylat | 0,92 |
| | Kontrollsumme | 100,00 |

### Beispiel 2

**Modifikation der Ölphase**

| chemische Bezeichnungen | Gew.-% (bevorzugt) | Gew.-% (typisch) |
|---|---|---|
| Isoamvllaurat | 8-16 | 15,00 |
| Cetearylalkohol | 15-30 | 24,00 |
| Glycerylcaprylat | 2-8 | 6,00 |
| Propylheptylcaprylat | 5-15 | 13,00 |
| Hexyldecanol | 5-10 | 7,00 |
| Hexyldecyllaurat | 5-10 | 7,00 |
| Burvrospermum Parkii Butter | 1-8 | 4,00 |
| Distärkephosphat | 5-15 | 9,00 |
| Glycerylstearatcitrat | 10-25 | 15,00 |
| 100,00 | | |

| | | |
|---|---|---|
| Cetearylalkohol = C16/18 | | |

### Beispiel 3

**Rezeptur Wasserphase**

| chemische Bezeichnungen | Gew.-% (bevorzugt) | Gew.-% (typisch) |
|---|---|---|
| Aqua | 65-75 | 73,50 |
| Natriumlauroylsarcosinat | 5-15 | 10,00 |
| Sorbitol | 2-8 | 3,00 |
| Glycerin | 5-20 | 13,00 |
| Dehvdroxanthan Gum | 0,1-1 | 0,50 |
| Milchsäure | ad pH 5,5 | |
| Kontrollsumme | | 100,00 |

### Beispiel 4

**Herstellung eines Emulsionskonzentrates**

| Bestandteile | Gew.-% (besonders bevorzugt) | Gew.-% (typisch) | Gew.-% (bevorzugt) |
|---|---|---|---|
| Wasserphase | 64 | 60 | 50-70 |
| Ölphase | 36 | 40 | 30-50 |
| Kontrollsumme | 100 | 100 | |

### Beispiel 5

**Aerosolrezeptur (Fußpflegeschaum)**

| Bestandteile | chemische Bezeichnung | Gew.-% | Anteile der Phasen |
|---|---|---|---|
| Wasser | Aqua | 25,512 | |
| Protelan LS 9011 | Natrium-Laurovlsarcosinat | 3,680 | |
| Karion F | Sorbitol | 1,288 | 35,53 |
| Glycerin | Glycerin | 4,775 | |
| Amaze XT | Dehvdroxanthan Gum | 0,276 | |
| Dermofeel sensolv | Isoamyllaurat | 2,760 | |
| Lanette 16 | Cetvlalkohol | 1,840 | |
| Dermosoft GMCY | Glycerylcaprvlat | 0,920 | |
| Cetiol sensoft | Propylheptylcaprylat | 2,760 | |
| Cetiol PGL | Hexyldecanol | 1,380 | |
| | Hexyldecyllaurat | 1,380 | 19,78 |
| Shea Butter | Burvrospermum Parkii Butter | 0,920 | |
| Mais PO4 PH "B" | Distärkephosphat | 1,840 | |
| Symbiomuls GC | Glycerylstearatecitrat | 2,900 | |
| | Cetearylalkohol | 2,900 | |
| | Glycerylcaprvlat | 0,179 | |
| Wasser | Aqua | 25,512 | |
| Avocadoöl | Persea Gratissima Oil | 1,840 | |
| Harnstoff | | 9,200 | |
| Milchsäure | | 0,083 | 44,69 |
| Natriumlactat | | 0,046 | |
| Microsilver BG | Silber | 0,009 | |
| Drivosol 27 | Isobutan, Propan, Butan | 8,000 | |
| | Kontrollsumme | 100,000 | 100,00 |

### Beispiel 6

**Fußschaum für Diabetiker (Aerosol)**

| Bestandteile | Gew.-% (bevorzugt)) | Gew.-% (typisch) |
|---|---|---|
| Emulsionskonzentrat (Bsp.4) | 45-60 | 55,00 |
| Aqua | ad 100 | 25,75 |
| Persea Gratissima Oil | 1-5 | 1,00 |
| Harnstoff | 2-20 | 10,00 |
| Milchsäure | 0,01-1,00 | 0,2 |
| Silber | 0,01-0,5 | 0,05 |
| Isobutan, Propan, Butan | 4-12 | 8,00 |
| Kontrollsumme | | 100,00 |

| | | |
|---|---|---|
| pH mit Milchsäure auf pH = 5,5 einstellen | | |

### Beispiel 7

**Körperschaum Anticellulits (Aerosol)**

| Bestandteile | Gew.-% (bevorzugt) | Gew.-% (typisch) |
|---|---|---|
| Emulsionskonzentrat (Bsp.4) | 45-60 | 55,00 |
| Aqua | ad 100 | 31,90 |
| Procontour™ | 2-8 | 5,00 |
| Milchsäure | 0,01-1,00 | 0,10 |
| Isobutan, Propan, Butan | 4-12 | 8,00 |
| Kontrollsumme | | 100,00 |

| | | |
|---|---|---|
| pH mit Milchsäure/Natriumlactat auf pH = 5,5 einstellen PROCONTOUR™: (Aqua, Alkohol, Lecithin, Coffein, Carnitin, Centella Asiatica Blatt-Extrakt, Kaliumphosphat, Coleus Barbatus Wurzel-Extrakt) | | |

ProContour™ wirkt nach Herstellerangaben gegen die tatsächlichen Zeichen der Cellulite. Eine In-vivo-Studie per Ultraschall ergab, dass ProContour™ die bei Cellulite in die Dermis hineinragenden Fettlobuli erheblich in deren Fläche, Breite sowie deren Länge und Höhe reduziert. Dies ist zurückzuführen auf den Synergismus dieses Wirkstoffes, der u. a. mit Coleus Forskohlii und Centella Asiatica Extrakten die gesamte Kaskade der bei Cellulite vorherrschenden Dysfunktionen der Mikrozirkulation, des Fettstoffwechsels und der Nachgiebigkeit des weiblichen Bindegewebes anspricht. Im Fettstoffwechsel stimuliert ProContour™ die β3-Adrenorezeptoren sowie das Enzym Adenylatcyclase. Beides erhöht die Konzentration von cAMP in den Adipozyten und steigert somit die Lipolyse. Weiterhin wird der Abtransport der Fettspaltprodukte unterstützt und die Mikrozirkulation erhöht. Die in diesem Fall essentielle Penetration wird von flexibel strukturierten Liposomen gewährleistet.

### Beispiel 8

**Wärmender Fußschaum (Aerosol)**

| Bestandteile | Gew.-% (bevorzugt) | Gew.-% (typisch) |
|---|---|---|
| Emulsionskonzentrat (Bsp.4) | 45-60 | 60,00 |
| Aqua | ad 100 | 29,90 |
| Corum 9235 | 0,5-5 | 2,00 |
| Milchsäure | 0,01-1,00 | 0,10 |
| Isobutan, Propan, Butan | 4-12 | 8,00 |
| Kontrollsumme | | 100,00 |

| | | |
|---|---|---|
| pH mit Milchsäure/Natriumlactat auf pH = 5,5 einstellen CORUM 9235: Vanillylbutylether | | |

CORUM 9235 ist nach Herstellerangaben ein effektiver Wärmewirkstoff, der eine sehr gute Hautverträglichkeit und ein äußerst geringes Irritationspotential aufweist. CORUM 9235 hat eine Vanillyl-Gruppe, die sich an die Neurorezeptoren in der Haut bindet und so ein langanhaltendes Wärmegefühl auf der Haut verursacht. CORUM 9235 ist besonders gut geeignet für Massagecremes und - öle und Winterformulierungen.

### Beispiel 9

**Selbstbräunungsschaum (Aerosol)**

| Bestandteile | Gew.-% (bevorzugt) | Gew.-% (typisch) |
|---|---|---|
| Emulsionskonzentrat (Bsp.4) | 45-60 | 55,00 |
| Aqua | ad 100 | 26,90 |
| CC Dihydroxyacetone DHA | 4-10 | 10,00 |
| Milchsäure | 0,01-1,00 | 0,10 |
| Isobutan, Propan, Butan | 4-12 | 8,00 |
| Kontrollsumme | | 100,00 |

| | | |
|---|---|---|
| pH mit Milchsäure auf pH = 4,5 einstellen CC Dihydroxyacetone (DHA): (Dihydroxyaceton, Aqua, Hydroxypropylcyclodextrin) | | |

Cyclosystemkomplexe (CC) sind Einkapselungen von Cyclodextrinen mit unterschiedlichen funktionellen Bestandteilen. Diese Cyclodextrinkomplexe sind bekannt für ihre zeitabhängige Freisetzung und ihre Schutzwirkung gegenüber den Wirkstoffen. Die zeitabhängige Freisetzung kann in einer erhöhten Wirksamkeit, einer lange anhaltenden Wirkung oder einem verringerten Irritationspotential resultieren, abhängig vom eingekapselten Wirkstoff.

### Beispiel 10

**Sonnenschutzschaum (Aerosol)**

| Bestandteile | Gew.-% (bevorzugt) | Gew.-% (typisch) |
|---|---|---|
| Emulsionskonzentrat (Bsp.4) | 45-60 | 55,00 |
| Aqua | ad 100 | 26,90 |
| Silica und Titandioxid¹⁾ | 5-15 | 10,00 |
| Milchsäure | 0,01-1,00 | 0,10 |
| Isobutan, Propan, Butan | 4-12 | 8,00 |
| Kontrollsumme | | 100,00 |

| | | |
|---|---|---|
| pH mit Milchsäure auf pH = 5,5 einstellen ¹⁾ Natural TiO2(ECOCERT) TiO2 eingekapselte Silica-Kügelchen (Natürlicher Non-Nano UV-Filter) | | |

### Beispiel 11

**Nachtcremeschaum (Aerosol)**

| Bestandteile | Gew.-% (bevorzugt) | Gew.-% (typisch) |
|---|---|---|
| Emulsionskonzentrat (Bsp.4) | 45-60 | 65,00 |
| Aqua | ad 100 | 22,90 |
| Butyrospermum Parkii Butter | 1-5 | 2,00 |
| Hippophae rhamnoides | 1-5 | 2,00 |
| Milchsäure | 0,01-1,00 | 0,10 |
| Isobutan, Propan, Butan | 4-12 | 8,00 |
| Kontrollsumme | | 100,00 |

| | | |
|---|---|---|
| pH mit Milchsäure auf pH = 5,5 einstellen Hippophae rhamnoides: Bio-Sanddorn-Fruchtfleischöl | | |

Die Kosmetik nutzt Bio-Sanddorn-Fruchtfleisch-Öl für emulgierte und ölige Haut- und Haarpräparate. Es wirkt besonders vorteilhaft auf trockene, sonnengerötete und strapazierte Haut. Die Carotinoide wirken als Radikalfänger, was der durch UV-Bestrahlung und Luftverschmutzung gestressten Haut zugute kommt.

### Beispiel 12

**Tagescremeschaum (Aerosol)**

| Bestandteile | Gew.-% (bevorzugt) | Gew.-% (typisch) |
|---|---|---|
| Emulsionskonzentrat (Bsp.4) | 45-60 | 60,00 |
| Aqua | ad 100 | 29,90 |
| AquaCacteen | 0,5-2,0 | 2,00 |
| Milchsäure | 0,01-1,00 | 0,10 |
| Isobutan, Propan, Butan | 4-12 | 8,00 |
| Kontrollsumme | | 100,00 |

| | | |
|---|---|---|
| pH mit Milchsäure auf pH = 5,5 einstellen AquaC acteen: Opuntia Ficus-Indica Stem-Extrakt und Glycerin und Wasser | | |

AquaCacteen ist ein hochgereinigtes Elixier aus organischem Kaktus (Opuntia Ficus-Indica). AquaCacteen blockiert die Freisetzung von Stressmarkern aus sensorischen Nervenzellen in der Haut und beruhigt gereizte Haut. Weiterhin besitzt es ausgezeichnete hydratisierende Eigenschaften aufgrund seines hohen Gehaltes an wasserbindenden Verbindungen.

### Beispiel 13

**Rasierschaum**

| Bestandteile | Gew.-% (bevorzugt) | Gew.-% (typisch) |
|---|---|---|
| Emulsionskonzentrat (Bsp.4) | 45-60 | 55,00 |
| Aqua | ad 100 | 29,00 |
| Natriumlauroylsarcosinat | 2-10 | 6,00 |
| AquaCacteen | 0,5-2,0 | 2,00 |
| Isobutan, Propan, Butan | 4-12 | 8,00 |
| Kontrollsumme | | 100,00 |

In den Beispielen 6-13 wurde das besonders bevorzugte Emulsionskonzentrat aus Beispiel 4 eingesetzt. Es kann jedoch bei diesen Beispielen jedes andere erfindungsgemäße Emulsionskonzentrat zum Einsatz kommen.

## Patentansprüche

1. pH-Wert-stabiles, keine freien Fettsäuren aufweisendes OW-Emulsionskonzentrat zur Herstellung von Hautkosmetika, umfassend
A) in der Ölphase
a) 4-12 Gew.-%, bezogen auf das Emulsionskonzentrat, einer Emulgatormischung auf Basis von Glycerylstearatcitrat, Cetearylalkohol und Glycerylcaprylat,
b) bis zu 2 Gew.-%, vorzugsweise 0,5-2 Gew.-%, bezogen auf das Emulsionskonzentrat, eines Caprylsäureesters,
c) wahlweise native und/oder synthetische kosmetische Öle,
d) wahlweise Fettalkohole mit 12-20 C-Atomen,
e) wahlweise Pflanzenbutter,
B) in der Wasserphase
a) 2-6 Gew.-%, bezogen auf das Emulsionskonzentrat, eines oder mehrerer schaumgebenden Tenside,
b) wahlweise Moisturizer,
c) Milchsäure und/oder deren Salze zur Einstellung des pH-Wertes des Emulsionskonzentrats auf 4,5 bis 6.

2. OW-Emulsionskonzentrat nach Anspruch 1, wobei das native kosmetische Öl aus der Gruppe gewählt ist, bestehend aus Jojobaöl, Sanddornfruchtfleischöl, Avocadoöl und Mandelöl, und das synthetische kosmetische Öl ein Fettsäureester ist, vorzugsweise gewählt aus der Gruppe, bestehend aus Cetylpalmitat, Isopropylmyristat, Isopropylpalmitat und Decyloleat.

3. OW-Emulsionskonzentrat nach Anspruch 1 und/oder 2, wobei die Pflanzenbutter Sheabutter ist.

4. OW-Emulsionskonzentrat nach mindestens einem der vorangehenden Ansprüche, wobei die schaumgebenden Tenside aus der Gruppe gewählt sind, bestehend aus Alkylsarcosinaten, Alkoylsarcosinaten, Alkylglutamaten, Alkoylglutamaten und deren Salzen.

5. OW-Emulsionskonzentrat nach mindestens einem der vorangehenden Ansprüche, wobei der Moisturizer gewählt ist aus der Gruppe, bestehend aus mehrwertigen Alkoholen und Zuckeralkoholen, vorzugsweise aus der Gruppe, bestehend aus Propylenglykol, Glycerin und Sorbitol.

6. Verfahren zur Herstellung eines OW-Emulsionskonzentrats nach mindestens einem der Ansprüche 1 bis 5, umfassend die Schritte:
i) Vorlegen der auf eine Temperatur von 30-50°C erwärmten und homogenisierten Ölphase
ii) Einrühren der eine Temperatur von 20-30°C aufweisenden Wasserphase, um durch Phasenumkehr ein OW-Emulsionskonzentrat zu bilden,
iii) Abkühlenlassen des OW-Emulsionskonzentrats unter Rühren auf Raumtemperatur.

7. Hautkosmetikum, umfassend ein OW-Emulsionskonzentrat nach mindestens einem der Ansprüche 1-5, ein Hautpflegeadditiv und/oder Hautschutzadditiv, zusätzlich Wasser zur Einstellung der gewünschten Konzentration der Bestandteile sowie gegebenenfalls mindestens ein Treibgas.

8. Hautkosmetikum nach Anspruch 7, wobei das Hautpflegeadditiv aus der Gruppe gewählt ist, bestehend aus rückfettenden Stoffen, Pro-Age-Mitteln, Selbstbräunungsmitteln, Anticellulitismitteln, Whiteningmitteln, Fußpflegemitteln, Hyaluronsäure, kühlenden oder wärmenden Mitteln, Stärken, phosphatveresterten Stärken auf Reis- und/oder Maisbasis, modifizierten Xanthangummis und hydratisierenden Mitteln.

9. Hautkosmetikum nach Anspruch 7 und/oder 8, wobei das Hautschutzadditiv aus der Gruppe gewählt ist, bestehend aus antibakteriellen Mitteln, antimykotischen Mitteln, Sonnenschutzmitteln auf organischer und/oder anorganischer Basis und Repellentwirkstoffen.

10. Hautkosmetikum nach mindestens einem der Ansprüche 7-9 für die Fußpflege, enthaltend hydratisierende Mittel, vorzugsweise Harnstoff, sowie strukturgebende Stoffe auf Basis von Cellulosederivaten.

11. Hautkosmetikum nach mindestens einem der Ansprüche 7-10 für ein Schaumaerosol, enthaltend als Treibgas Dimethylether und/oder Kohlenwasserstofftreibmittel, sowie gegebenenfalls Stickstoffund/oder CO₂ zur Druckstufeneinstellung.

12. Hautkosmetikum nach Anspruch 11 für ein sprühbares, aufschäumendes Aerosol, abgefüllt in einer 2-Kammer [bag-on-valve (BOV)] Aerosoldruckdose oder einer 3-Kammer [bag-in-bag-on-valve (BBOV)] Aerosoldruckdose.

## Claims

1. An OW emulsion concentrate having a stable pH value and containing no free fatty acids for producing skin cosmetics, comprising
A) in the oil phase
a) 4-12 % by weight, in relation to the emulsion concentrate, of an emulsifier mixture based on glyceryl stearate citrate, cetearyl alcohol and glyceryl caprylate,
b) up to 2 % by weight, preferably 0.5-2 % by weight, in relation to the emulsion concentrate, of a caprylic acid ester,
c) optionally native and/or synthetic cosmetic oils,
d) optionally fatty alcohols having 12-20 C atoms,
e) optionally vegetable butter,
B) in the water phase
a) 2-6 % by weight, in relation to the emulsion concentrate, of one or more foaming surfactants,
b) optionally moisturiser,
c) lactic acid and/or salts thereof for setting the pH value of the emulsion concentrate to 4.5 to 6.

2. The OW emulsion concentrate according to Claim 1, wherein the native cosmetic oil is selected from the group consisting of jojoba oil, sea buckthorn berry oil, avocado oil and almond oil, and the synthetic cosmetic oil is a fatty acid ester, preferably selected from the group consisting of cetyl palmitate, isopropyl myristate, isopropyl palmitate and decyl oleate.

3. The OW emulsion concentrate according to Claim 1 and/or 2, wherein the vegetable butter is Shea butter

4. The OW emulsion concentrate according to at least one of the preceding claims, wherein the foaming surfactants are selected from the group consisting of alkyl sarcosinates, alkoyl sarcosinates, alkyl glutamates, alkoyl glutamates, and salts thereof.

5. The OW emulsion concentrate according to at least one of the preceding claims, wherein the moisturiser is selected from the group consisting of polyvalent alcohols and sugar alcohols, preferably from the group consisting of propylene glycol, glycerol and sorbitol.

6. A method for preparing an OW emulsion concentrate according to at least one of Claims 1 to 5, comprising the steps of:
i) providing the oil phase, which is heated to a temperature of 30-50°C and is homogenised,
ii) stirring in the water phase, which has a temperature of 20-30°C, in order to form an OW emulsion concentrate by means of phase inversion,
iii) allowing the OW emulsion concentrate to cool to room temperature while stirring.

7. A skin cosmetic, comprising an OW emulsion concentrate according to at least one of Claims 1-5, a skin care additive and/or skin protection additive, additionally water for setting the desired concentration of the components, and optionally at least one propellant gas.

8. The skin cosmetic according to Claim 7, wherein the skin care additive is selected from the group consisting of regreasing substances, pro-age agents, self-tanning agents, anti-cellulite agents, whitening agents, foot care agents, hyaluronic acid, cooling or warming agents, starches, phosphate-esterified starches based on rice and/or maize, modified xanthan gums, and hydrating agents.

9. The skin cosmetic according to Claim 7 and/or 8, wherein the skin protection additive is selected from the group consisting of antibacterial agents, antimycotic agents, sun protection agents on organic and/or inorganic basis, and repellent active agents.

10. The skin cosmetic according to at least one of Claims 7-9 for foot care, containing hydrating agents, preferably urea, and structuring substances based on cellulose derivatives.

11. The skin cosmetic according to at least one of Claims 7-10 for a foam aerosol, containing dimethyl ether and/or hydrocarbon propellants as propellant gas, and also optionally nitrogen and/or CO₂ for pressure level adjustment.

12. The skin cosmetic according to Claim 11 for a sprayable aerosol that foams up, filled in a 2-chamber [bag-on-valve (BOV)] pressurised aerosol can or a 3-chamber [bag-in-bag-on-valve (BBOV)] pressurised aerosol can.

## Revendications

1. Concentré en émulsion EH d'acides gras libres ayant une valeur de pH stable pour la préparation de dermo-cosmétiques, comprenant
A) dans la phase huileuse
a) 4 à 12 % en poids, relatif au concentré en émulsion, d'un mélange d'émulsifiant sur la base de glycérylstéaratcitrate, de cétéarylalcool, et de glycérylcaprylate,
b) jusqu'à 2 % en poids, préférablement 0,5 à 2 % en poids, relatif au concentré en émulsion, d'un ester d'acide caprylique,
c) facultativement des huiles cosmétiques natives et/ou synthétiques,
d) facultativement des alcools gras ayant 12 à 20 atomes de carbone,
e) facultativement du beurre végétal,
B) dans la phase aqueuse
a) 2 à 6 % en poids, relatif au concentré d'émulsion, d'un ou de plusieurs agents tensioactifs moussants,
b) facultativement de l'humidifiant,
c) de l'acide lactique et/ou ses sels pour le réglage de la valeur pH du concentré en émulsion à 4,5 à 6.

2. Concentré en émulsion EH selon la revendication 1, dans lequel l'huile cosmétique native est choisie parmi le groupe constitué par l'huile de jojoba, l'huile de pulpe de baies d'argousier, l'huile d'avocats et l'huile d'amandes, et l'huile cosmétique synthétique est un ester d'acide gras, préférablement choisi parmi le groupe constitué par le palmitate de cétyle, le myristate d'isopropyle, le palmitate d'isopropyle et l'oléate de décyle.

3. Concentré en émulsion EH selon la revendication 1 et/ou 2, dans lequel le beurre végétal est du beurre du karité.

4. Concentré en émulsion EH selon au moins une des revendications précédentes, dans lequel les agents tensioactifs moussants sont choisi parmi le groupe constitué par les sarcosinates d'alkyle, les sarcosinates d'alcoyle, les glutamates d'alkyle, les glutamates d'alcoyle et leur sels.

5. Concentré en émulsion EH selon au moins une des revendications précédentes, dans lequel l'humidifiant est choisi parmi le groupe constitué par les alcools et alcools de sucre polyvalents, préférablement parmi le groupe constitué par le glycol de propylène, la glycérine, et le sorbitol.

6. Procédé pour la préparation d'un concentré en émulsion EH selon au moins une des revendications 1 à 5, comprenant les étapes:
i) la présentation de la phase huileuse chauffée à une température de 30 à 50°C et homogénéisée
ii) l'ajout de la phase aqueuse ayant une température de 20 à 30°C, afin de former, par inversion de phase, un concentré en émulsion EH,
iii) le laisser refroidir du concentré en émulsion EH en remuant jusqu'à température ambiante.

7. Cosmétique dermatologique comprenant un concentré en émulsion EH selon au moins une des revendications 1 à 5, un additif de soin de peau et/ou un additif de protection de peau, en plus de l'eau pour le réglage de la concentration désirée des composants ainsi que facultativement au moins un gaz propulseur.

8. Cosmétique dermatologique selon la revendication 7, dans lequel l'additif de soin de peau est choisi parmi le groupe constitué par les matières relipidiantes, les moyens pro-âge, les agents auto-bronzant, les agents anti-cellulitiques, les agents de blanchiment, les agents des soin de pieds, l'acide hyaluronique, les agents refroidissant ou chauffant, les amidons, les amidons estérifiés au phosphate sur base de riz et/ou de maïs, les gommes de xanthane modifiés et les agents hydratants.

9. Cosmétique dermatologique selon la revendication 7 et/ou 8, dans lequel l'additif de protection de peau est choisi parmi le groupe constitué par les agents antibactériens, les agents antimycotiques, les agents protégeant du soleil sur base organique et/ou inorganique et les agents répulsifs.

10. Cosmétique dermatologique selon au moins une des revendications 7 à 9 pour le soin de pieds, comprenant des agents hydratants, préférablement de l'urée, ainsi que des substances structurantes sur la base de dérivés de cellulose.

11. Cosmétique dermatologique selon au moins une des revendications 7 à 10 pour aérosol moussant, comprenant en tant que gaz de propulsion de l'éther méthylique et/ou un agent de propulsion à base d'hydrocarbure, ainsi que facultativement de l'hydrogène et/ou du CO₂ pour le réglage des étapes de pression.

12. Cosmétique dermatologique selon la revendication 11 pour un aérosol moussant, capable d'être pulvérisé, remplit dans un récipient à pression pour aérosol à deux chambres [bag-on-valve (BOV)] ou dans un récipient à pression pour aérosol à trois chambres [bag-in-bag-on-valve (BBOV)].
